# EUROPEAN PATENT SPECIFICATION

(11) **EP 2 016 188 B1**
(45) Date of publication and mention of the grant of the patent: **21.10.2015**
(21) Application number: 06771406.3
(22) Date of filing: 27.05.2006
(51) Int. Cl.: C12Q 1/68, C12P 19/34

(54) **METHODS FOR DETECTION OF METHYLATED DNA**
VERFAHREN ZUM NACHWEIS METHYLIERTER DNA
PROCÉDÉS POUR LA DÉTECTION D'ADN MÉTHYLÉ

(30) Priority: 24.04.2006 US 409766
(43) Date of publication of application: 21.01.2009
(73) Proprietor: Jia, Xiyu, Newport Beach CA 92660 (US)
(72) Inventor: Jia, Xiyu, Newport Beach CA 92660 (US)
(74) Representative: Potter Clarkson LLP
(86) International application number: PCT/US2006/020618
(87) International publication number: WO 2007/123553

(56) References cited:
- WO-A-2004/067545
- WO-A-2005/054502
- WO-A1-2004/067545
- US-A- 5 804 375
- US-A1- 2003 148 290
- US-A1- 2003 180 748
- US-A1- 2005 153 308
- US-B2- 6 960 436
- GRUNAU C ET AL: "Bisulfite genomic sequencing: systematic investigation of critical experimental parameters" NUCLEIC ACIDS RESEARCH, OXFORD UNIVERSITY PRESS, SURREY, GB, vol. 29, no. 13, 1 January 2001 (2001-01-01), pages e65-1, XP002241346 ISSN: 0305-1048
- PAULIN RICHARD ET AL: "Urea improves efficiency of bisulphite-mediated sequencing of 5'-methylcytosine in genomic DNA" NUCLEIC ACIDS RESEARCH, OXFORD UNIVERSITY PRESS, SURREY, GB, vol. 26, no. 21, 1 November 1998 (1998-11-01), pages 5009-5010, XP002210104 ISSN: 0305-1048
- HERMAN J G ET AL: "Methylation-specific PCR: a novel PCR assay for methylation status of CpG islands." PROCEEDINGS OF THE NATIONAL ACADEMY OF SCIENCES OF THE UNITED STATES OF AMERICA 3 SEP 1996, vol. 93, no. 18, 3 September 1996 (1996-09-03), pages 9821-9826, XP002542477 ISSN: 0027-8424

### SEQUENCE LISTING

<110> Jia, Xiyu
<120> Methods For Detection of Methylated DNA
<130> ZRC-7
<140> 60/691,539
   <141> 2005-06-17
<160> 8
<170> PatentIn version 3.3
<210>. 1
   <211> 14
   <212> DNA
   <213> Artificial Sequence
<220>
   <223> m5c at position 8
<220>
   <221> modified_base
   <222> (8) .. (8)
   <223> m5c
<400> 1
   ataactgcgt aacc 14
<210> 2
   <211> 14
   <212> DNA
   <213> Artificial Sequence
<220>
   <223> bisulfite modified
<220>
   <221> misc_difference
   <222> (5) .. (14)
   <223> c at positions 5, 13, and 14 are converted to u
   and act as t in molecular assays.
<400> 2
   ataattgcgt aatt 14
<210> 3
   <211> 15
   <212> DNA
   <213> Artificial Sequence
<220>
   <223> m5c at position 5
<220>
   <221> modified_base
   <222> (5) .. (5)
   <223> m5c
<400> 3
   gttgcgctca ctgcc 15
<210> 4
   <211> 15
   <212> DNA
   <213> Artificial Sequence
<220>
   <223> bisulfite modified
<220>
   <221> misc_feature
   <222> (7) .. (15)
   <223> c at positions 7, 9, 11, 14, and 15 are converted
   to u and act as t in molecular assays
<400> 4
   gttgcgttta ttgtt 15
<210> 5
   <211> 22
   <212> DNA
   <213> Artificial Sequence
<220>
   <223> Sense
<220>
   <221> misc_feature
   <222> (1) .. (22)
   <223> Sense
<400> 5
   gatgcgtaag gagaaaatac cg 22
<210> 6
   <211> 21
   <212> DNA
   <213> Artificial Sequence
<220>
   <223> Antisense
<220>
   <221> misc_feature
   <222> (1) .. (21)
   <223> Antisense
<400> 6
   cgcctctccc cgcgcgttgg c 21
<210> 7
   <211> 22
   <212> DNA
   <213> Artificial Sequence
<220>
   <223> Sense
<220>
   <221> misc_feature
   <222> (1) .. (22)
   <223> sense
<220>
   <221> misc_feature
   <222> (1) .. (22)
   <223> Sense
<400> 7
   gatgtgtaag gagaaaatat tg 22
<210> 8
   <211> 21
   <212> DNA
   <213> Artificial Sequence
<220>
   <223> Antisense
<220>
   <221> misc_feature
   <222> (1) .. (21)
   <223> Antisense
<400> 8
   cacctctccc cacacattaa c 21

## Description

### CLAIM OF PRIORITY

This application claims priority to U.S. Provisional Application Serial No. 60/691,539, filed on June 17, 2005.

### FIELD OF THE INVENTION

The invention relates to the regulation of gene expression and more specifically to improved methods for determining the methylation status of specific DNA templates using the bisulfite method of methylation analysis.

### BACKGROUND

DNA methylation is a ubiquitous process in prokaryotes and eukaryotes. In prokaryotes DNA methylation protects an organism's own DNA from restriction enzymes produced to digest foreign DNA. In higher, eukaryotes DNA methylation functions in the regulation of gene expression (Costello and Plass, 2001). Aberrant methylation is widespread in cancer and may be among the earliest changes to occur during oncogenesis (Stirzaker, 1997). DNA methylation has also been shown to play a central role in gene imprinting, embryonic development, X-chromosome gene-silencing, and cell-cycle regulation. In many plants and animals, DNA methylation consists of the addition of a methyl group to the fifth position carbon of the cytosine pyrimidine ring via a methyltransferase enzyme (Adams, 1995). The majority of DNA methylation in mammals occurs in 5'-CpG-3' dinucleotides but other methylation patterns have been described. Remarkably, about 80-percent of all 5'-CpG-3' dinucleotides found in mammalian genomes are methylated. Of the remaining 20-percent unmethylated dinucleotides, most are found within gene promoters and first exons.

The ability to detect and quantify DNA methylation efficiently and accurately has become essential for the study of cancer, gene expression, genetic diseases, and other areas of biology. To date, a number of methods have been developed to measure DNA methylation including, high-performance capillary electrophoresis (Fraga, M.F., 2000), methylation-sensitive arbitrarily primed PCR (Gonzalgo, M.L. (1997), and the bisulfite method coupled to chemical denaturation (Frommer, M., 1992).

Most commonly used methods are variations of the bisulfite methods, in which DNA is treated with bisulfite, causing unmethylated cytosines to be converted into uracil while methylated cytosines are relatively unaffected. In the standard bisulfite protocol, using chemical denaturation, typically with a strong base, and bisulfite conversion. The bisulfite-modified DNA can be amplified by PCR and the resulting PCR products are either analyzed by DNA sequencing or restriction endonuclease digestion. U.S Pat. Nos. 5,786,146, 6,017,704, 6,200,756, and 6,265,171 (and their foreign equivalents) describe methylation specific PCR (MSP) detection methods for methylated nucleic acids. The methylation profile of the DNA segment is then determined by comparing the sequence of the bisulfite-treated DNA to that of the untreated DNA. Each of these patent documents is incorporated by references.

Recent methods disclosed in U.S. patent application US 2004/0219538 have sought to remove chromatographic steps used to purify by employing a dilution/precipitation regime. However, these recent methods continue to rely on alkali denaturation, subject to the typical degradation and recovery problems. In addition precipitation in lieu of chromatography (to clean up the bisulfite conversion reaction) involves significantly more processing time.

Methods disclosed in U.S. patent application US 2005/0079527 employ size exclusion. devices to facilitate the final isolation of the bisulfite converted template DNA. However, this methodology suffers similarly from significantly longer processing time. Use of size exclusion chromatography also requires elution of the sample in significantly larger volumes, which is usually undesirable.

An important step and remaining technological hurdle in methylation detection is the efficient denaturation of the DNA template prior to the separate step of bisulfite modification. The standard technique of chemical denaturation is limited in denaturation efficiency and is time consuming. Unnecessary sample handling and the requirement for alkali denaturation results in significant degradation of the template DNA, which adversely affects essentially all down stream methylation analysis. Improving the quality, reliability, and speed of methylation detection has clear implications in terms of the early detection and diagnosis of animal diseases, disorders, and for analysis of methylation changes in biological systems.

### REFERENCES

Each of the following documents, as well as documents cited in the description are considered relevant prior art: WO2004/067545: suggests in a method for bisulfite treatment of DNA a separate alkaline DNA denaturation step. Bisulfite conversion is performed for 1.5-3.5h between 70°C-90°C.

US6960436: discloses a method for preparing a DNA template for methylation analysis comprising heat denaturation of double stranded DNA at variable temperatures. "For high molecular weight DNA, the denaturation temperature is generally greater than 90°C". A cyclic protocol consisting of variable denaturation temperatures is suggested.

The bisulfite conversion is consisting of two steps, the sulfonation and deamination for which cyclic conditions with changing temperatures are suggested. A preferred variant comprises a change of temperature from 4°C (10min) to 50°C (20min). It is mentioned that said chemical conversion can be performed in the presence of denaturing solvents.

US2005/0153308: performs bisulfite conversion at a temperature between 40°C-60°C and even suggests that a denaturation step is not required.

WO2005/054502: suggests use of guanidinium hydrogen sulfite without prior denaturation of DNA. Deamination of DNA is performed for 2h at 80°C.

US5804375: suggests a "typical heat denaturation involves temperatures ranging from about 80°C to 105°C for times ranging from a few seconds to minutes". Bisulfite conversion of DNA is not addressed.
Costello, J.F. and Plass. C. (2001) J. Med. Genet: 285-303.
Stirzaker, C. (1997) Cancer Res. 57:2229-37.
Adams, R.L. (1995) Bioessays 17: 139-45
Fraga, M.F. (2000) Electrophoresis 21:2990-94.
Fraga, M.F. and Esteller M. (2002) BioTechniques Vol. 33, No. 3, 632-649.
Gonzalgo, M.L. (1997) Cancer Res. 57: 594-99.
Frommer, M. (1992) Proc. Natl. Acad. Sci. U.S.A. 89: 9821-26.
Herman J, (1996) Proc. Natl. Acad. Sci. U.S.A. 93:1827-31.
Yanisch-Perron, et al. (1985), Gene 33, (1), 109-119.
Sambrook and Russell (2001) Molecular Cloning, Cold Spring Harbor Laboratory Press:1.64
U.S Pat. No. 5,786,146
U.S Pat. No. 6,017,704
U.S Pat. No. 6,200,756
U.S Pat. No. 6,265,171
U.S. Pat. App. 2004/0219539 A1
U.S. Pat. App. 2005/0079527 A1

### SUMMARY OF THE INVENTION

The invention provides a method for preparing DNA template for DNA methylation analysis. An important feature of the invention is the combined denaturation and bisulfite conversion, where cytosines are sulfonated and deaminated, using a high temperature. Some embodiments of the invention are coupled to in-column desulfonation of sample DNA for high yield recovery in a small elution volume. In a preferred embodiment, the temperature of denaturation is 96°C to 100°C. In a particular embodiment, the high temperature is about 96°C.

In some embodiments, this high temperature incubation is the first of several different temperature incubations, some of which are described herein. In some embodiments, the first high temperature incubation is followed by an additional incubation at a temperature of about 30°C to about 92°C. In particular embodiments the additional incubation is at one or more temperatures of from about 37° to about 55°C.

Some embodiments include a further or second additional incubation at a temperature from about 50°C to about 70°C. In one embodiment this temperatures of from about 37° to about 55°C.

In some embodiments, the combined denaturation and bisulfite conversion reaction is performed with the aid of a thermal cycling device; however, the present invention is not limited by this methodology or technology. Thermal cycling according to the following regime is used in some embodiments, but the invention is not limited to this particular scheme, program, or thermal cycling methodology:
1. 96-98°C for 10 minutes.
2. 50-53°C for 30 minutes.
3. 50-53°C for 6 minutes.
4. 37°C for 30 minutes.
5. 4°C for storage.

Steps 3-4 can be repeated for about 8 cycles.

In other embodiments thermal cycling according to the following regime may improve results.
1. 98°C for 10 minutes.
2. 64°C for 2.5 hours.
3. 4° for up to 20 hours storage.

In some embodiments of the invention, the combined denaturation and bisulfite conversion is performed in the absence of added strong base to accomplish denaturation of the DNA template, including but not limited to sodium, potassium, lithium, cesium, and/or magnesium hydroxide. In embodiments of the present invention the pH of the combined denaturation and bisulfite conversion is about 7.0 or less, or even about 5.5 or less

The combined denaturation and bisulfite conversion of the sample DNA may be performed in the presence or absence of one or more chemical agents that stimulate the conversion such as guanidine salts, diethyl triamine, spermine (polyamines), urea, glycerol, formamide, dimethylformamide (DMF), quinol, hydroquinol, 8-hydroxy quinone, or other like agents. These agents may be reducing or denaturing in character, but function to increase the efficiency of the bisulfite conversion reaction. In a particular embodiment of the invention, the agent is the chemical agent DMF. In preferred embodiments DMF is used at concentrations from about 0.2 to about 40% of the bisulfite conversion solution, or final sample volume.

The combined denaturation, bisulfite conversion, and desulfonation of the sample DNA may be performed in the presence or absence of a surfactant, such as sodium dodecylsulfate (SDS), TWEEN-20, NONIDET P-40, or other ionic and/or nonionic detergents, at a concentration sufficient to increase DNA denaturation efficiency at predetermined thermal denaturation conditions.

In some embodiments of the invention, combined denaturation and bisulfite conversion are followed by coupled and rapid desulfonation with the DNA adsorbed to a solid matrix. In a particular embodiment the solid matrix is the resin in a column. In preferred embodiments the resin is prepared from glass fiber, or like material, known in the art (*e.g*., WHATMAN: type C and F glass fibers). The column may be adapted to assist in the handling of the adsorbed. An advantage of the column is that time consuming and often inefficient precipitation steps are avoided. The contemplated formats include columns suitable for use in standard centrifuges, microfuges (spin-columns), vacuum manifolds, or low or high pressure (HPLC, HPCE) modulated columns, all of which are commonly known in the art. The columns can be in a 96-well plate or strip format. In other embodiments columns in 384-well formats may be used. Such desulfonation is referred to herein as coupled in-column desulfonation.

One embodiment of the invention is a method for preparing denatured, bisulfite converted, and desulfonated DNA template in a coupled process, the method comprising (a) performing combined denaturation and bisulfite conversion of the DNA by incubating at a temperature of from 96° to 100°C; (b) adsorbing the sulfonated DNA to a solid matrix; (c) desulphonating the adsorbed DNA on a solid matrix at a temperature from about 20°C to about 96°C; and (d) eluting the desulfonated DNA from the solid matrix.

In many embodiments, the elution is into a small volume of aqueous solution. The time of incubation at a given temperature may be adjusted to optimize denaturation, bisulfite conversion, and desulfonation. In a particular embodiment of the invention, the solid matrix is in a column, or like device. In some embodiments the solid matrix comprises beads or membranes. In-column desulfonation is accomplished by passing a basic solution over the column.

As noted above and elsewhere, the combined single-reaction denaturation and bisulfite modification may be performed in the presence or absence of one or more chemical reducing or denaturing agent that stimulates the conversion (sulfonation/deamination). The presence of added strong base to facilitate denaturation before bisulfite reagent is added is not required. The use of heat alone in the presence of bisulfite and buffer mediates the denaturation and conversion in a single reaction saving significant time and also increasing overall efficiency.

Another aspect of embodiments of the present invention is the use of a column for adsorbing DNA just subsequent to the combined thermal denaturation and bisulfite conversion modification at a high temperature. The column comprises a resin for binding or immobilizing the DNA during the coupled process. The column further allows efficient elution into small volumes of aqueous solutions.

### BRIEF DESCRIPTION OF THE DRAWINGS

**Figure 1****:** Depicts embodiments of methylated DNA preparation.
**Figure 2****:** Depicts the sequence of template DNA prepared using embodiments of the present invention. The top DNA sequence represents the original DNA with a methylated C^{m}pG at position 5. The bottom DNA sequence is the same as the top after bisulfite conversion.
**Figure 3****:** Depicts the results of thermal denaturation time course for DNA templates.
**Figure 4****:** Depicts the results of the bisulfite conversion time course.
**Figure 5****:** Depicts the results of changes in temperature incubation regimes in specific embodiments.

### DETAILED DESCRIPTION OF THE INVENTION

### Definitions

Prior to describing the invention in detail, the following definitions are provided. Other technical and scientific terms are to be afforded their ordinary meaning as used in the art.

*Solid matrix*: Beads, column matrix material (resin), membrane filter material, or other particulate matter to which DNA can be adsorbed for coupled desulfonation of denatured bisulfite converted (sulfonated/deaminated) DNA template. The solid matrix material should be stable for the limited time at high temperatures described herein. The solid matrix should also be stable in the presence of strong bases or denaturing agents, depending on the particular embodiment of the invention. Silica-based solid matrices are only one example. Others include, but are not limited to silica particles; silica resins; silica gel; hydrophobic-interaction silica resin; silica-embedded filters; magnetic silica particles; macroporous silica gel; and combinations thereof (Sambrook and Russel, 2001). In addition, modified silica materials and ion exchange resins may be used, either alone or in combinations, in the form of hybrid resins. Other mixed ion-exchange/adsorption chromatographic techniques have been commonly used in nucleic acid purification procedures and are suitable for use in additional embodiments. These include anion exchange (DEAE) and other hydrophobic interaction-based compounds. Preferred solid matrices simplify the handling of DNA samples. Particularly useful solid matrices are silica beads and membranes columns comprising a silica-based resin used in a column.

A preferred column is a spin-column, which is used in embodiments described herein and in a commercially available product from ZYMO RESEARCH CORP. The column includes a narrow, tapered neck where silica resin material (or matrix) is contained. The configuration promotes efficient combined adsorption and desulfonation of the bisulfited converted DNA template for elution into a small volume. In other embodiments, silica-based resin multi-well-plates are used, including, but not limited to 96-wells, or 384-wells, each well functioning as a column with a narrow tapered configuration that promotes efficient DNA template adsorption and desulfonation of DNA and elution into a small volume (ZYMO RESERCH CORP.). Such columns include a unique, tapered chamber bed for containing silica resin. These other embodiments contemplate use of similarly designed plates capable of use with, 96-well, 384-well, or greater number formats in automated high through-put analysis. A feature of these columns and plates is low-volume elution for allow the user to obtain concentrated DNA template at a suitable concentration. A typical volume is between about 4 µl to about 50 µl. In particular embodiments the volume is about 4 µl to about 20 µl or even about 8 µl to about 12 µl. This provides for concentrated samples amenable to sensitive downstream methylation analysis.

*Binding agent*: A solution for promoting the binding of denatured, bisulfite-converted DNA to a solid matrix. The preferred binding agent is a guanidine buffer of about 6 M final concentration. Other chaotropic agents include, but are not limited to, iodide, perchlortate, and trichloroacetate. Other DNA binding agents are known in the art.

*Denaturation efficiency* and *Bisulfite conversion efficiency*: Use of this terminology is consistent with its use in nucleic acid hybridization, methylation, and PCR technologies.

*Denaturation:* Generally refers to the efficiency of separating methylated or unmethylated double-stranded DNA. (dsDNA) into single stranded DNA (ssDNA), usually using heat, a strong base (*e.g*., NaOH, KOH, LiOH, or similar), and/or a chemical denaturant or chaotropic agent (*e.g*., guanidinium, urea, other chaotropic agents, SDS, detergent or surfactant, etc.). As used herein, denaturation is efficient when at least 80%, at least 90%, at least 95%, at least 98%, or even at least 99% of the dsDNA present in a sample is converted to ssDNA.

In the presence of a bisulfite, reagent, the bisulfite conversion is combined with denaturation. Bisulfite conversioin efficiency refers to the efficiency of conversion of cytosine (C) to uracil (U) by bisulfite (*e.g.*, sodium bisulfite, magnesium bisulfite, and other bisulfite salts) after later desulfonation treatment (see, *e.g.*, below). As used herein, denaturation is efficient when over 95% of the available unmethylated cytosine residues present in a sample are converted into uracil. The overall denaturation efficiency and bisulfite conversion efficency may be adjusted by one skilled in the art by varying incubation time and temperature.

*Desulfonation*: Refers to C to U conversion via deamination of the sulfonated cytosine Desulfonation may be performed by the addition of strong base including the base in a buffer (*e.g.*, 0.25 M NaOH, 80% ethanol), such that the pH of the final desulfonation solution is basic or alkaline, for example about 10 or more. Desulfonation may be carried out at a variety of temperatures, preferably between about 20°C to about 96°C, and most preferably between about 20° to about 30°C for suitable, incubation times to accomplish desulfonation. As used herein, desulfonation is efficient when over 95% of the available unmethylated cytosine residues present in a sample are converted into uracil.

*Temperature* and *high temperature*: Use of this terminology is consistent with incubation at a single temperature or more than one temperature within a specified range or about a specified temperature. For example, "a temperature between temperatures X and Y," includes a single incubation temperature between X and Y as well as multiple temperatures between X and Y. Multiple temperatures should be construed to encompass cycling between temperatures within the specified range.

A high temperature for combined denaturation and bisulfite conversion is from 96° to 100°C. Preferred individual high temperatures include about 96°C, about 97°C, about 98°C, and about 99°C.

Temperature-cycling devices may be used to facilitate incubation at multiple incubation temperatures, or for any of the incubations described. Additional or intermittent incubations that are outside the specified temperature range, but which do not defeat the purpose of the incubation, do not circumvent the scope of the invention.

*DNA*: DNA encompasses methylated and unmethylated DNA and single and doublestranded DNA, unless the description or context indicates or requires otherwise. The DNA may be mixed with other known or unknown components, *e.g.*, in a sample or clinical specimen. The DNA may be chemically or enzymatically modified to the extent that it does not interfere with the underlying principles of the invention. The DNA may be genomic DNA, epimeric DNA, synthetic DNA, or DNA from other sources.

*Strong base*: As used herein, a strong base is one of sufficient strength to denature DNA. Examples include alkali metal hydroxides, such as NaOH and KOH. Note that the term refers to the final conditions under which DNA is incubated. The mere presence of base, which is neutralized by one or more other components, does not satisfy the definition. As used herein, the final pH of a strong base solution is preferably about 10 or higher.

*Bisulfite Reagents*: As used herein bisulfite reagents refer to saturated solutions of sodium bisulfite, although other bisulfite salts, such as lithium, potassium, ammonium, magnesium, manganese, and calcium, may substitute in some embodiments of the present invention. Bisulfite reagents may include sodium hydroxide (NaOH), or other alkali hydroxides, *e.g.*, at concentrations of OH⁻ from about 0.3 M to about 0.4,M. as well as the antioxidant hydroquinone and reducing agents, or other stimulants of the bisulfite conversion (*i.e.*, formation of a sulfonate adduct). In some embodiments, the concentration of bisulfite ion is from about 0.8 M to about 2.5 M final concentrations. In other embodiments the bisulfite ion may be present at a concentration of about 1.0 M to about 2.0 M. The pH of the bisulfite reagent is from about pH from about 4.8 to about 5.5 (not a strong base as defined herein). The bisulfite reagent is light sensitive and thus it is preferred to conduct the denaturation and bisulfite conversion in the dark.

*Sample*: A clinical, forensic, or other biological specimen comprising or suspected of comprising DNA, from as small as a single cell, a few cells; to larger quantities of cells or cellular samples, or small or large tissue sources.

*Stimulating Agents:* Refer to chemical agents that stimulate the bisulfite reaction and include, but are not limited to, guanidine salts, diethyl triamine, spermine (polyamines), urea, glycerol, formamide, dimethylformamide (DMF), quinol, hydroquinone, 8-hydroxyquinone methoxyamine, or, related agents.

*Unification*: As used herein purification refers to the chromatographic isolation of template DNA from all solutions in a sufficiently pure form suitable for sensitive methylation analysis after the combined denaturation, bisulfite conversion, and coupled in-column desulfonation, followed by elution in a small volume of aqueous solution, from about 4 µl to about 100 µl, in other embodiments the elution is about 4 µl to about 50 µl, and in still other embodiments about 4 µl to about 20 µl, and in still other embodiments in about 8 µl to about 12 µl.

### DESCRIPTION OF THE INVENTION

The following description is of the best mode presently contemplated for practicing the invention. The description should not be construed as limiting but is made merely for the purpose of describing the general principles of the invention. The scope of the invention should be ascertained with reference to the description and the knowledge of those skilled in the art.

The invention is an improvement over existing methods for performing the bisulfite conversion method for modifying DNA for determination of the methylation state. In particular, the invention includes a method for preparing denatured, bisulfite converted, and desulfonated template DNA using combined thermal denaturation and bisulfite conversion at higher than normal temperatures. This method combines/integrates the previously separate steps of DNA denaturtion and bisulfite conversion, providing a more efficient one-step process for denaturation and efficient bisulfite modification (sulfonate adduct formation).

The denatured and converted DNA may additionally be adsorbed to a solid matrix for efficient, coupled desulfonation and/or to assist in handling and recovery of the DNA. The resulting DNA is efficiently denatured, bisulfite converted, and desulphonated, while being minimally degraded. The DNA may be eluted in a small volume for downstream methylation analysis.

The classical method for methylation detection using bisulfite treatment includes two separate and distinct steps. The first step is to denature double stranded DNA into single stranded DNA (ssDNA). The second step is treatment of the ssDNA with sodium bisulfite under conditions whereby cytosine (C) is converted to uracil (U) but where methylated cytosine (*i.e.*, 5-methylcytosine; C^{m}) is relatively non-reactive. Conversion entails sulfonation of the cytosine at position 6 with concurrent addition of a hydrogen at position 5, followed by desulfonation via deamination under basic conditions, which completes conversion to uracil.

The uracil in the treated DNA behaves like thymine (T) in most biological reaction (such as methylation specific PCR (MSP), sequencing analysis and restriction endonuclease digestion, HPCE, or HPLC, among other assays, while the 5-methylcytosine continues to behave as a cytosine (Herman, J, 1996). For example, the sequence of SEQ ID NO: 1 (5'-A T A A C T G C^{m} G T A A C C-3'), which contains a 5-methylcytosine as the 8^{th} nucleotide (nt) will have unmethylated cytosine nucleotides converted to uracil by by the bisulfite reaction (e.g. cytosines at positions 5, 13, and 14,). This sequence will behave as SEQ ID NO: 2 (5'-AT-AA**T**TGC^{m}GTAA**TT**-3') in most biological assays.

For the first step, the traditional method uses chemical, alkali-based denaturation to convert dsDNA into ssDNA to improve accessibility of the cytosine residues. Mostly, sodium hydroxide (NaOH) is used for this purpose. NaOH is added to the DNA, and incubated for certain periods of time to make the DNA become single stranded (*e.g*., chemical denaturation), next, bisulfite is added to the denatured DNA to start the bisulfite conversion process.

The present invention uses heat to replace traditional chemical denaturation and integrates denaturation and bisulfite conversion into a combined and continuous process for increased efficiency, automation and ease of manipulation. This combined process does not require the use of a strongly basic solution alkali for the purpose of denaturation. The present invention may be coupled to the rapid, in-column desulfonation methodology, which is described for certain embodiments.

An important feature of the present invention is that the combined thermal denaturation of the DNA and bisulfite conversion produces template suitable for the most sensitive downstream methylation analysis. Without being limited to a theory, it is believed that the use of high temperature, or thermal denaturation, is more efficient than conventional chemical methods for denaturation used in the bisulfite method. Moreover, the higher than normal temperatures promote efficient bisulfite conversion (*i.e.*, conversion via sulfonation of ssDNA template). This streamlines the procedure as well as increases the efficiency of conversion. It also reduces loss of DNA via degradation or from other causes.

In certain embodiments, this combined denaturation/bisulfite conversion is coupled to adsorption of the resulting DNA to a solid matrix to facilitate desulfonation by increasing DNA concentration and optimizing DNA conformational/topology for desulfonation. When a DNA molecule is adsorbed on the matrix, the ssDNA segments are thought to be present as loops that extend outward, or as contiguous segments adsorbed on the surface. Accessibility and desulfonation is believed to be enhanced compared to desulfonation (C to U) carried out in solution.

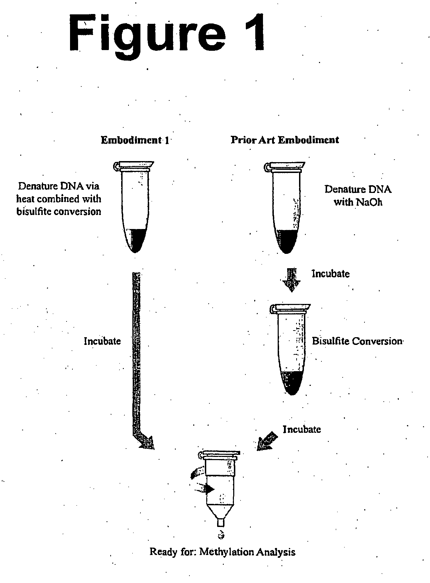

A comparison of an embodiment of the present invention with the prior art is shown schematically in Figure 1 (See Embodiment: 1 one step preferred Method, 2 traditional two-step method). The preferred embodiment and other embodiments of the present invention share combined denaturation and bisulfite conversion coupled to innovative in-column desulphonation methodology that eliminates cumbersome DNA precipitation steps and maximizes recovery in a small elution volume. Other embodiments entail simply the combined denaturation and bisulfite conversion. Embodiments of the present invention provide researchers consistent recovery of template DNA.

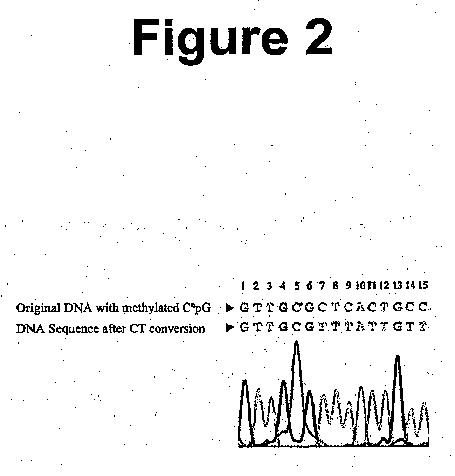

The sequencing results of DNA samples amplified by PCR either with or without bisulfite conversion are shown (See figure 2). Samples that were bisulfite, treated were processed using the preferred embodiment of the present invention (See example 3). The original input plasmid DNA represented by the top sequence contains a methylated cytosine at nucleotide position 5 (C^{m}pG)(SEQ ID NO: 3). The methylated cytosine is resistant to bisulfite conversion. The bottom sequence is the same DNA after being processed by a preferred embodiment after bisulfite conversion of the DNA (SEQ ID NO: 4). Note the methylated cytosine at position 5 remained intact while unmethylated cytosines (*e.g.*, positions 7, 9, 11, 14, and 15) were efficiently converted into uracil and detected as thymine.

The invention has been streamlined for high yield recovery of DNA following DNA bisulfite conversion and desulfonication in a small volume. The use of a high temperature for denaturation combined with bisulfite conversion (Figure 1 Embodiment 1; Example 3 Protocol) replaces traditional chemical denaturation, performed by a first step of incubating the DNA in the presence of NaOH followed by addition of bisulfite in a second step, which is most common of bisulfite conversion methods (Figure 1: Embodiment 2; Example 4 protocol). Both methods are shown schematically.

Kits exemplifying the invention have been designed to minimize template degradation, loss of DNA during treatment and clean-up, and to provide complete conversion of unmethylated cytosines. Recovered DNA is ideal for PCR amplification for downstream analyses including restriction endonuclease digestion, sequencing, microarray analysis, among other procedures employing, high performance capillary electrophoresis (HPCE), or high pressure liquidchromatography (HPLC).

### Thermal Denaturation Time Course Experiment

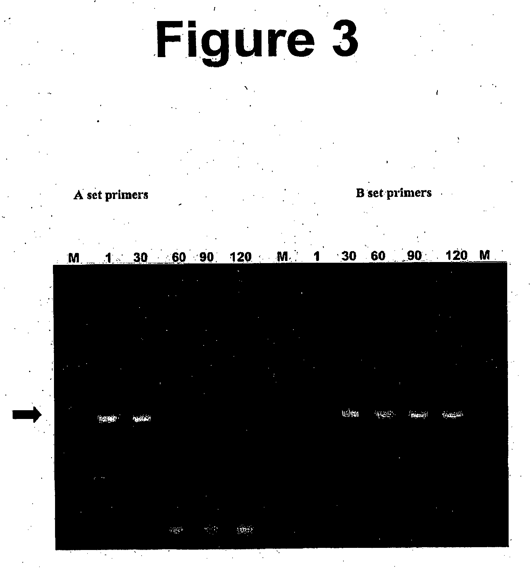

A time course experiment was conducted to examine the kinetics for thermal denaturation of plasmid DNA at 96°C. After denaturation by heat in the presence of bisulfite reagent (see Example 3), for various times, all samples were incubated at 50°C for about 4 hours. The samples were then processed using standard DNA isolation procedures, PCR, and agarose gel electrophoresis, by methods known in the art. The results are shown in Figure 3.

Plasmid DNA samples of about 5 pg were incubated for different times at high temperature to determine the parameters of using heat alone to denature DNA for combined bisulfite mediated conversion (pUC19, Genbank Acession No. L09137; Yanisch-Perron, et al. 1985). The unconverted and converted templates were monitored by primer set A (SEQ ID NOs: 5 and 6) and set B SEQ ID NOs: 7 and 8) respectively. These primers are listed yin Example 1. Referring to Figure 3, the numbers on the top indicate the various incubation times at 96°C (1, 30, 60, 90, and 120 seconds; the, M indicates the 100 bp DNA marker from NEBL). Individual samples were generally processed according to the protocol detailed in Example 3. The PCR products migrate between 400-500 bp in the 1.0% agarose gel.

Referring to Figure 3, at the 1 and 30-second time points, there was still original unconverted plasmid DNA present which could be amplified by primer set A. The minimal time for denaturation was 60 seconds when unconverted template was minimized and converted template was easily detected. However, longer incubation times of up to 120 seconds did not appear to cause degradation of the template DNA

The results showed that combined thermal denaturation and bisulfite conversion function efficiently, obviating the need for separate chemical denaturation and bisulfite conversion steps. Generally, the use of combined heat and Bisulfite conversion can be adapted for use with any DNA template and is not limited to a particular source.

### Bisulfite Conversion Time Course Experiment

The incubation time for combined heat denaturation and bisulfite conversion of the same plasmid DNA was tested in the following experiments. Samples were denatured at 96°C for 3 minutes in the presence of bisulfite reagent. Individual samples were generally processed according to the protocol detailed in Example 3. Samples were then incubated for various times at 50°C to complete the bisulfite conversion process (See Figure 3: 2 minutes to 8 hours in duplicate; The capital M indicates 100 bp DNA marker)(NEBL). After each sample was taken out at the indicated time point, the samples were stored at 4°C for later process when all the samples were collected. For example, the 2 minutes incubation time point samples were stored at 4°C for 8 hours before they were processed.

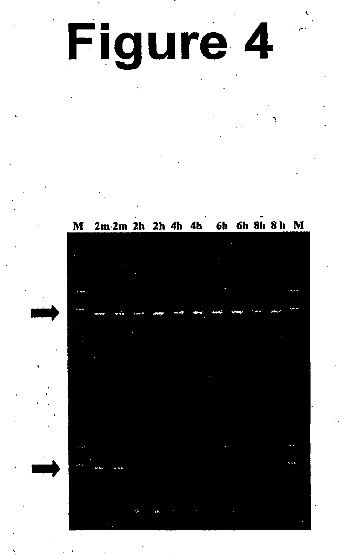

This experiment revealed that the bisulfite conversion by this methodology was dependent on the temperature (See Figure 4). The top panel indicates the results of primer set B for converted template. The specific PCR product is represented by the 470 bp fragment migrating in the middle of the agarose gel (See arrow: top and bottom panels). The bottom panel indicates the results of primers set A for unconverted template. The results further indicated that the DNA templates were not efficiently converted after 2 minutes 50°C incubation (bottom panel primer A set; arrow). Incubation between 2-6 hours had no apparent effect on the template conversion efficiency. When incubation was extended up to 8 hours the bisulfite conversion was decreased slightly as assayed by the PCR products (compared to 2-6 hours incubation time points). The optimal time for bisulfite conversion at 50°C is between 4-6 hours.

The observation that the bisulfite conversion was heat dependent led to the idea of using combined heat and bisulfite conversion in a single reaction without added base, thereby avoiding the need for separate denaturation and bisulfate steps. Since it has been reported that bisulfite conversion, or sulfonation of cytosine, is favored at low reaction temperatures and low pH (optimum at about pH 5.0), the observation that utilizing a high temperature for combined denaturation and bisulfite conversion is unexpected. In addition to saving time, the present invention provides efficient conversion and recovery, particularly when Combined with coupled solid matrix (*e.g.*, in-column) desulfonation.

### Bisulfite Conversion at Different Temperatures.

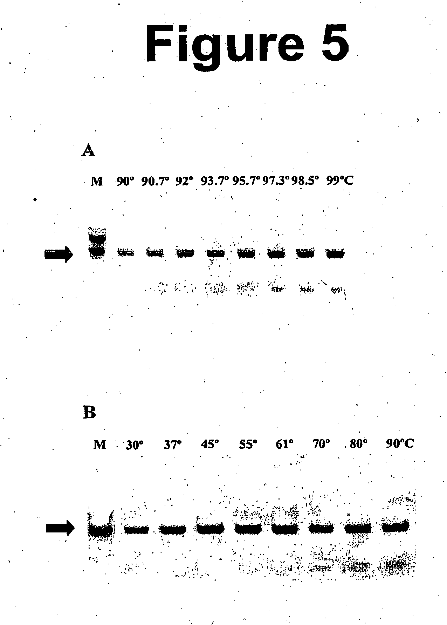

Varying temperature for the combined denaturation and bisulfite conversion was explored to define ranges that would be effective. The plasmid DNA samples were further processed by in-column desulfonation and reaction clean up according to the protocol outlined in example 3, and as described above except, for the various changes to temperature. The 470 bp plasmid (pUC19) was amplified with the primer Set B that specifically amplifies bisulfite converted template. Samples of about 5 pg of plasmid DNA were incubated at the first temperature for about 10 minutes in a gradient at indicated temperatures, 90°, 90.7°, 92°, 93.7°, 95.7°, 97.3°, 98.5°, and 99°C (See figure 5A, M indicates molecular weight marker, NEBL). Here, the samples were not further incubated at an additional temperature, but would be according to some embodiments of the invention.

The stained gel shows the relative amount of amplified, bisulfite-converted sample plasmid DNA obtained following high temperature denaturation at the indicated temperatures. The maximum amount was obtained at the temperature of about 97.3°C, although temperatures of about 95.7° to about 99°C produced similar results. This experiment indicates that denaturation under the conditions for combined denaturation and bisulfite conversion occurs at a single incubation from about 90° to about 100°C, with the preferred temperature centering about 98°C.

Some embodiments of the invention include one or more additional incubations at different temperatures. Such incubations improve the efficiency of bisulfite conversion.

To determine the preferred temperature range for additional incubations, the samples of plasmid DNA were first incubated for 10 minutes at a high temperature of 98°C, then incubated at an additional temperature of 30°; 37°, 45°, 55°, 61°, 70°, 80°, or 90°C for 30 minutes (See figure 5B). Additional temperature allows control of the overall degree of bisulfite conversion and can be varied in specific embodiments to accomplish desired levels. In this experiment bisulfite converted DNA was evident across the temperature range from about 30° to about 90°C. The maximum amount of bisulfite converted DNA was seen at about 45° to about 61°C.

The combined thermal denaturation and bisulfite conversion need not be coupled to the Desulfonation process. However, the coupled in-column desulfonation on a solid matrix increases the efficiency of template recovery and minimizes degradation in addition to the rapid purification of the template.

The standard technique of chemical denaturation is limited in denaturation efficiency and time consuming. Improving DNA template denaturation efficiency will enhance the overall bisulfite conversion and thus increase the ability to detect methylation variations among DNA templates. Identifying such variations is the target of basic clinical research in the identification of inheritable diseases such as forms of cancer. Improving the efficiency of methylation detection will reduce costs and aid in the analysis and diagnosis of human disease.

While the invention has been described by means of specific embodiments and applications thereof, it is understood that numerous modifications and variations could be made thereto by those skilled in the art.

### EXAMPLES

### Reagents and Methodology

### Example 1: Diagnostic Primers

The primer pair A (SEQ. ID. NO: 4 and 5) are used to amplify a 470 bp DNA fragment from the pUC19 plasmid by PCR (**Genbank Acession No. L09137**), when unmethylated cytosine (C) is not converted to uracil (U) (see Figure 3).

The primer pair B (SEQ. ID. NO: 6 and 7) are used amplify a corresponding 470 bp DNA fragment from pUC19 by PCR when C is converted to U.

Set A:
Seq I.D. No. 5:
   208-229 nt, Plus sense to amplify original sequence.
      5'-gatgcgtaaggagaaaataccg-3'
Seq I.D. No. 6:
   648-667 nt. Minus sense to amplify original sequence.
      5'-cgcctctccccgcgcgttggc-3'

Set B:
Seq I.D. No. 7:
   B1: Plus sense, primers for use after C to U/T conversion.
      5'-gatgtgtaaggagaaaatattg-3'
Seq I.D. No. 8:
   B2: Minus sense, primer after C to U/T conversion
      5'-cacctctccccacacattaac-3'

Bisulfite conversion can be monitored using primer pair sets A and B. Primer set A detects unconverted DNA, whereas primer set B detects bisulfite-converted DNA where base C has been converted to U (see Figure 1-3). Primers were synthesized, prepared by commercial sources (IDT DNA Inc., Iowa), and all PCR methods and agarose electrophoresis were performed as known in the art.

### Example 2: Reagents, Solutions, and buffers

**Bisulfite Reagent (Solution):** The bisulfite conversion reagent for approximately 10 reactions is a solid mixture of about 475 mg sodium metabisulfite (Na₂S₂O₅, 1.9 M) and 0.5 mg hydroquinone (4.6 µM). It is recognized that sodium bisulfite and similar bisulfite salts may be used at concentrations from about 0.2 M to about 1.0 M, or about 1.0 M to about 2.0 M. In addition, the antioxidant hydroquinone may be omitted, or alternately used from about 3.0 µM to about 10 µM, or about 4 µM to about 5 µM. The dry chemicals are dissolved by adding 900 µl H₂0, 50 µl of 50% DMF, and 300 µl of 2 M NaOH. The solution is mixed until the chemicals are completely dissolved. Vortexing or shaking is recommended. Many compositions of bisulfite conversion reagent are known in the art. It is not thought that minor variations in the concentrations or inclusion of specific additional chemical components, other than bisulfite, significantly affect conversion efficiency.

The resulting solution is about pH 5.1 and comprises: 1.9 M Na₂S₂O₅; 4.5 uM hydroquinone; 0.4 M NaOH; and 100 mM DMF. It is normal to see trace amounts of undissolved reagent since the bisulfite conversion solution is near saturation. The dissolved bisulfite conversion solution is typically used immediately, but may be stored in the dark at about 20°-30°C for short periods, or until use for up to one week at - 20°C. If the solution is stored at -20°C upon thawing it should be mixed thoroughly before use. The bisulfite conversion solution is light sensitive and thus light exposure should be minimized.
**Dissolving Buffer:** 50% DMF.
**Dilution Buffer:** 2 M NaOH.
**Desulfonation Solution:** 0.25 M NaOH and 80% ethanol, final concentration.
**Binding Buffer:** 6 M Guanidine-HCl.
**DNA Wash Buffer:** 80% ethanol and 10 mM Tris-HCl, pH 7.5, final concentration.
**Elution Buffer:** Pure N₂0, or TE, pH 8.5 (10 mM Tris; 1 mM EDTA).

### Example 3: Combined Thermal Denaturation and Bisulfite Conversion: Coupled to In-Column Desulfonation.

The bisulfite conversion and desulfonaton (C-U) of DNA is accomplished using the following protocol. The Reaction volumes may be scaled as required (see embodiment 1, Figure 1). **Step 1:** A sample of 130 µl of the nearly saturated bisulfite solution from Example 2 is added to about 20 µl of DNA sample in a 250 ul tube (suitable for PCR reactions). For example: 4 µl DNA sample, 16 µl H₂O, and 130 µl bisulfite conversion solution constitutes a standard reaction, though modifications to account for DNA sample volume can readily be made. The DNA sample may contain from about 500 pg to about 2.0 µg of DNA template, and preferably between about 200 ng to about 500 ng. The sample may also contain about 500 ng of salmon sperm DNA or other non-interfering DNA carrier. The sample is mixed by hand by flicking the tube, or other convenient means. The sample is placed in a thermal-cycler device and subjected to either of the following temperature programs. For some samples the alternate temperature regime parameters may yield improved results, while other temperature combinations may also yield further improvements.

### Step 2: Thermal-Cycle Regime.

1. 96-98°C for 10 minutes.
2. 50-53°C for 30 minutes.
3. 50-53°C for 6 minutes.
4. 37°C for 30 minutes.
5. 4°C for storage (optional).

**Note: Elements** 3-4 should be repeated for about 8 cycles.

### Step2: Alternate Thermal-Cycle Regime.

1. 98°C for 10 minutes.
2. 64°C for 2.5 hours.
3. 4°C for up to 20 hours.

Step 3: Following completion of the temperature program, 600 µl of a 6 M guanidine-HCl solution *(i.e.,* binding buffer) is added. **Step 4:** The resulting solution is applied directly onto a column, for example a spin-column positioned onto a collection tube. **Step 5:** The column and collection tube are centrifuged in a microfuge at the maximum setting (> 10,000 x g) for about 30 seconds. **Step 6:** A sample of 200 µl of desulfonation solution is added onto the column and let stand incubating at room temperature (RT) for about 15-20 minutes (RT: about 20 ° to 30°C ± 5°C). The column is centrifuged a second time at the maximum setting for about 30 seconds. **Step 7:** A 200 µl sample of wash buffer is added onto the column and centrifuged a second time at the maximum setting for about 30 seconds. As the collection tube has limited capacity (usually about 800 µl) it should be emptied when ever necessary to prevent contamination of the column contents by the flow-through. **Step 8:** Elution into a small volume is performed by addition of about 2 to about 20 µl, preferably about 4 to about 12 µl, or about 4 to about 8 µl, or about 4 µl to about 100 µl of elution buffer. The eluted DNA is suitable for immediate analysis or can be stored at or below -20°C for later use. Typically, 2 to about 4 µl of eluted DNA is suitable for use in standard methylation analysis such as PCR, or other procedures (see Figures 1-4). The entire process can be completed rapidly, (e.g., in about 3 to about 3 and a half hours).

For DNA samples in volumes greater than about 20 µl, an adjustment for the bisulfite conversion reagent is recommended. The amount of water is decreased to 100 µl for each 10 µl increase in DNA sample volume. For example, for a 40 µl DNA sample 700 µl of water is added to make the bisulfite conversion reagent. For this protocol the suggested maximum DNA sample to be used for each conversion reaction is about 50 µl. It is recognized that many variations and modifications to the above mentioned protocol can be made to account for different samples, desired increase, or decrease bisulfite conversion efficiencies, or to otherwise tailor the combined methodology as desired by one skilled in the art.

### Example 4: Standard Traditional Two-Step Methodology (chemical, NaOH, denaturation followed by bisulfite conversion)

The bisulfite modification and conversion (C to U) of DNA for an embodiment representative of the prior art, for comparison purposes to embodiments of the present invention, is accomplished by the following protocol. The reaction volumes may be scaled as required (See embodiment 2, Figure 1). The bisulfite reagent solution used in this protocol is about pH 5.3 and consists of: 1.9 M Na₂S₂O₅; 4.5 uM hydroquinone; and about 0.2 M NaOH.

**Step 1:** A sample or 5 µl of the dilution buffer (2M NaOH) is added to the DNA sample and the total volume adjusted to 50 µl with sterile water. For example: 14 µl of DNA sample, 5 µl of dilution buffer (2 M NaOH), and 31 µl of water form a standard chemical denaturation reaction. **Step 2:** The sample is incubated at 37°C for about 15 minutes. The combination of steps 1 and 2 constitute the chemical denaturation reaction started with addition of strong base (NaOH). **Step 3:** A 100 µl aliquot of the bisulfite conversion solution is added to the DNA sample in a 250 ul tube (suitable for PCR reactions). This begins the bisulfite conversion procedure of the denatured DNA (ssDNA). The DNA sample should contain between about 200 pg to about 2 µg DNA template. Carrier DNA of about 500 ng of salmon sperm DNA may be added. The sample is mixed by hand by flicking the tube, or other convenient means. **Step 4:** The sample is incubated at 50°C for about 12-16 hours to allow for sufficient conversion. **Step 5:** The sample is placed on ice for abut 10 minutes to stop the reaction. **Step 6:** An aliquot of 400 µl of binding buffer (guanidine-HCL) is added and mixed by pipetting up and down several times to prepare the sample for purification.

**Step 7:** The mixed sample is loaded onto a column, for example a spin-column, and placed onto a collection tube. **Step 8:** The column and collection tube are centrifuged in a microfuge at the maximum setting (> 10,000 x g) for about 30 seconds. Note: the collection tube has limited capacity, usually about 800 µl and should be emptied when ever necessary to prevent contamination of the column contents by the flow-through. **Step 9:** An aliquot of 200 µl of wash buffer (10 mM Tris pH 7.5, 80% ethanol) is added and the sample is centrifuged a second time at the maximum setting for about 30 seconds. **Step 10:** An aliquot of 200 µl of desulfonation solution (0.25 M NaOH, 80% ethanol) is added onto the column and incubated at RT for about 15 minutes, followed by centrifugation at the maximum setting for 30 seconds. **Step 11:** A second aliquot of 200 µl of wash buffer is added and the sample is centrifuged a second time at the maximum setting for about 30 seconds. **Step 12:** Elution into a small volume is performed by addition of about 2 to about 4 µl, or 4 to about 20 µl, or alternatively about 8 to about 12 µl of elution buffer. The eluted DNA is suitable for immediate methylation analysis or can be stored at or below -20°C for later use. Typically, the eluted DNA is suitable for use in standard methylation analysis such as PCR, or other sensitive procedures (see Figures 1-3). The entire process can be completed in about 13-16 hours. The bisulfite conversion reaction consists of two main and separated steps: (1) The chemical denaturation of the DNA template with NaOH; (2) addition of bisulfite for conversion of the denatured template DNA.

### Example 5: 96-Well Format for Combined Thermal Denaturation and Bisulfite Conversion

The buffers and solutions are essentially the same as those described in example 3. Changes made in regards to volumes or amounts are indicated below. The sodium bisulfite reagent is prepared essentially the same as described above except for scaling for more sample volumes. The bisulfite reagent is identical to that described in example 3. The following procedure is an adaptation of the protocol in example 3 (embodiment 1) to a high-through put 96-well format.

**Step 1:** A 5-20 µl of DNA samples is added to wells of a 96-well PCR plate. The total volume in each well is adjusted to 20 µl with sterile water. For example: 14 µl DNA sample, 6 µl water is added. **Step 2:** A 130 µl aliquot of the bisulfite solution is added to the DNA sample in each well (the 96-well plate should be suitable for thermal cycling). The DNA sample should contain from about 200 pg to about 2.0 µg DNA template. Carrier DNA of about 500 ng of salmon sperm DNA may be added. The sample is mixed by pipetting, or other convenient means. The plate should be sealed by an appropriate means such as a cover tape or foil to prevent sample loss during incubation. **Step 3:** The sample is places in a thermal-cycler device and subjected to the temperature program as stated in example 3. **Step 4:** An aliquot of 400 µl, or 600 µl of binding buffer (guanidine-HCL) is added and mixed by pippetting up and down several times. **Step 5:** Transfer the samples from the **PCR plate** (above) to the wells of the 96-well filter plate (Smaller volume shallow-well Silicon-A filter plate; larger volume for the Deep-well Zymo-Spin 1-96 filter plate). Different filter plates are commercially available and may be used with embodiments of this invention (ZYMO RESEARCH CORP.: Deep-well Zymo-Spin 1-96 filter plate, or shallow-well Silicon-A Filter plates are two examples). **Step 6:** The 96 well filter plate is placed over a collection plate and put into a centrifuge capable of handling plates and centrifuged at about 2,500 to about 5,000 rpm for about 5 minutes. The flow through is discarded from the collection plates. Stronger centrifugal forces are best for thorough removal of buffer from the plates. **Step 7:** About 400 µl of wash buffer (80% ethanol) is added to each well and the plate is centrifuged at about 2,500 to about 5,000 rpm for about 5 minutes. The collection plate can be emptied whenever necessary to prevent contamination of the filter plate if the volume of buffer is more than 800 µl. **Step 8:** About 200 µl of desulfonation buffer (0.25 M NaOH; 80% ethanol) is added and incubated at room temperature, or about 20° to 30°C for 15 minutes. After incubation the plate is centrifuged at about 2,500 to about 5,000 rpm for 5 minutes. **Step 9:** About 400 µl of wash buffer (80% ethanol, 10 mM Tris, pH 7.5) and centrifuged at about 2,500 to about 5,000 rpm for 5 minutes. This wash step is repeated but is centrifuged for a longer time of 10 minutes to ensure removal of the entire wash buffer. **Step 10:** The filter plate is placed onto an elution plate. About 15-30 µl of elution buffer or water, or TE (10 mM Tris, 1.0 mM EDTA, pH 7.5) is added to each well. The plate is centrifuged at about 2,500 to about 5,000 rpm for 5 minutes to elute the DNA template. The entire process can be completed in about 4 hours.

Embodiments of the present invention are adaptable to 96-well, 384-well, or greater number formats outlined in the above example. In these high-throughput embodiments the increased efficiency and coupled one-step denaturation and bisulfite conversion would result in significant advantages.

## Claims

1. A method for preparing DNA template for DNA methylation analysis, the method comprising incubating DNA template in the presence of a bisulfite reagent at a temperature between 96°C to 100°C for about 10 minutes to produce the combined denaturation and bisulfite conversion of the DNA template.

2. The method of claim 1 further comprising:
adsorbing the DNA to a solid matrix;
desulfonating the adsorbed DNA; and
eluting the desulfonated DNA from the solid matrix.

3. The method of claim 1, wherein the denaturation and bisulfite conversion of the sample DNA is performed in the presence of a chemical reducing agent.

4. The method of claim 1, wherein the denaturation and bisulfite conversion of the sample DNA is performed in the presence of a chemical denaturation reagent.

5. The method of claim 3, wherein the chemical reducing agent is selected from the consisting of hydroquinone and 8-hydroxyquinone.

6. The method of claim 4, wherein the chemical denaturing agent is selected from the group consisting of DMF, glycerol, and urea.

7. The method of claim 2, wherein the eluting is in a volume from 4µl to 20µl of an aqueous solution.

8. The method of claim 1, further comprising incubating the DNA template at a temperature between 30°C to 92°C.

9. The method of claim 8 further comprising incubating the DNA template for between 2 to 6 hours.

10. The method of Claim 1, wherein the temperature of incubation is between 98°C to 100°C.

11. The method of Claim 7, wherein the eluted aqueous solution has a volume from 8µl to 12µl.

12. The method of Claim 1, wherein the temperature of incubation is between 96°C to 98°C.

13. The method of Claim 1, wherein the temperature of incubation is about 98°C.

## Patentansprüche

1. Verfahren zur Herstellung eines DNA-Templats für die DNA-Methylierungsanalyse, wobei das Verfahren das Inkubieren eines DNA-Templats in Gegenwart eines Bisulfitreagenzes bei einer Temperatur zwischen 96°C und 100°C für etwa 10 Minuten umfasst, um auf diese Weise eine kombinierte Denaturierung und Bisulfitumwandlung des DNA-Templats zu bewirken.

2. Verfahren nach Anspruch 1, weiterhin umfassend:
Adsorbieren der DNA an eine feste Matrix;
Desulfonieren der adsorbierten DNA; und
Eluieren der desulfonierten DNA von der festen Matrix.

3. Verfahren nach Anspruch 1, wobei die Denaturierung und Bisulfitumwandlung der Proben-DNA in Gegenwart eines chemischen Reduktionsmittels durchgeführt wird.

4. Verfahren nach Anspruch 1, wobei die Denaturierung und Bisulfitumwandlung der Proben-DNA in Gegenwart eines chemischen Denaturierungsmittels durchgeführt wird.

5. Verfahren nach Anspruch 3, wobei das chemische Reduktionsmittel aus der Gruppe bestehend aus Hydrochinon und 8-Hydroxychinon ausgewählt ist.

6. Verfahren nach Anspruch 4, wobei das chemische Denaturierungsmittel aus der Gruppe bestehend aus DMF, Glycerin und Harnstoff ausgewählt ist.

7. Verfahren nach Anspruch 2, wobei das Eluieren in einem Volumen von 4 µl bis 20 µl einer wässrigen Lösung erfolgt.

8. Verfahren nach Anspruch 1, weiterhin umfassend das Inkubieren des DNA-Templats bei einer Temperatur von zwischen 30°C und 92°C.

9. Verfahren nach Anspruch 8, weiterhin umfassend das Inkubieren des DNA-Templats für 2 bis 6 Stunden.

10. Verfahren nach Anspruch 1, wobei die Inkubationstemperatur zwischen 98°C und 100°C beträgt.

11. Verfahren nach Anspruch 7, wobei die eluierte wässrige Lösung ein Volumen von 8 µl bis 12 µl besitzt.

12. Verfahren nach Anspruch 1, wobei die Inkubationstemperatur zwischen 96°C und 98°C beträgt.

13. Verfahren nach Anspruch 1, wobei die Inkubationstemperatur etwa 98°C beträgt.

## Revendications

1. Procédé de préparation une matrice d'ADN pour une analyse de méthylation d'ADN, le procédé comprenant l'incubation d'une matrice d'ADN en présence d'un réactif bisulfite à une température entre 96°C et 100°C pendant environ 10 minutes pour produire la dénaturation et la conversion du bisulfite de manière combinée de la matrice d'ADN.

2. Procédé selon la revendication 1 comprenant en outre :
l'adsorption de l'ADN sur une matrice solide ;
la désulfonation de l'ADN adsorbé ; et
l'élution de l'ADN désulfoné à partir de la matrice solide.

3. Procédé selon la revendication 1, dans lequel la dénaturation et la conversion du bisulfite de l'ADN de l'échantillon sont effectuées en présence d'un agent réducteur chimique.

4. Procédé selon la revendication 1, dans lequel la dénaturation et la conversion du bisulfite de l'ADN de l'échantillon sont effectuées en présence d'un réactif de dénaturation chimique.

5. Procédé selon la revendication 3, dans lequel l'agent de réduction chimique est choisi dans le groupe constitué d'hydroquinone et 8-hydroxyquinone.

6. Procédé selon la revendication 4, dans lequel l'agent de dénaturation chimique est choisi dans le groupe constitué de DMF, glycérol, et urée.

7. Procédé selon la revendication 2, dans lequel l'élution est réalisée dans un volume de 4 µl à 20 µl d'une solution aqueuse.

8. Procédé selon la revendication 1, comprenant en outre l'incubation de la matrice d'ADN à une température entre 30°C et 92°C.

9. Procédé selon la revendication 8, comprenant en outre l'incubation de la matrice d'ADN pendant 2 à 6 heures.

10. Procédé selon la revendication 1, dans lequel la température d'incubation est située entre 98°C et 100°C.

11. Procédé selon la revendication 7, dans lequel la solution aqueuse éluée a un volume de 8 µl à 12 µl.

12. Procédé selon la revendication 1, dans lequel la température d'incubation est située entre 96°C et 98°C.

13. Procédé selon la revendication 1, dans lequel la température d'incubation est d'environ 98°C.
